Europäisches Patentamt

European Patent Office

Office européen des brevets

(11) Veröffentlichungsnummer: **0 008 412**
**B1**

(12) **EUROPÄISCHE PATENTSCHRIFT**

(21) Anmeldenummer: **79102875.6**

(22) Anmeldetag: **09.08.79**

(51) Int. Cl.³: **C 07 C 69/44,**
**C 07 C 67/38, //**
**C 07 C 69/533**

(54) Verfahren zur Herstellung von Butandicarbonsäureestern.

(30) Priorität: **21.08.78 DE 2836518**

(43) Veröffentlichungstag der Anmeldung:
**05.03.80 Patentblatt 80/5**

(45) Bekanntmachung des Hinweises auf die Patenterteilung:
**28.01.81 Patentblatt 81/4**

(84) Benannte Vertragsstaaten:
**BE CH DE FR GB IT NL**

(56) Entgegenhaltungen:
**FR - A - 2 384 738**

(73) Patentinhaber: **BASF Aktiengesellschaft**
**Carl-Bosch-Strasse 38**
**D-6700 Ludwigshafen (DE)**

(72) Erfinder: **Platz, Rolf, Dr.**
**Hansastrasse 5**
**D-6800 Mannheim 1 (DE)**
(72) Erfinder: **Kummer, Rudolf, Dr.**
**Kreuzstrasse 6**
**D-6710 Frankenthal 5 (DE)**
(72) Erfinder: **Schneider, Heinz-Walter, Dr.**
**Bruesseler Ring 43**
**D - 6700 Ludwigshafen (DE)**

Courier Press, Leamington Spa, England.

Verfahren zur Herstellung von Butandicarbonsäureestern

Gegenstand der Erfindung ist ein Verfahren zur Herstellung von Butandicarbonsäureestern, bei dem man Butadien oder Butadien enthaltende Kohlenwasserstoffgemische mit Kohlenmonoxid und niederen Alkanolen in Gegenwart von tertiären Stickstoffbasen und Kobaltcarbonyl bei Temperaturen von 80 bis 150°C unter erhöhtem Druck umsetzt und den so erhaltenen Pentensäureester mit Kohlenmonoxid und niederen Alkanolen bei Temperaturen von 140 bis 200°C unter erhöhtem Druck weiter zu Butandicarbonsäureestern umsetzt, wobei die Kobalt enthaltende Katalysatorlösung im Kreis geführt wird.

Aus Bull. Chem. Soc. Japan, 46, 1973, Seiten 524 ff. ist ein zweistufiges Verfahren zur Herstellung von Adipinsäureestern aus Butadien bekannt, wobei man Butadien zunächst mit Kohlenmonoxid und Alkanolen in Gegenwart von Kobaltcarbonyl und Stickstoffbasen, wie Pyridin oder Isochinolin, umsetzt, und ohne den Katalysator zu entfernen in einer darauffolgenden Stufe den entstandenen Pentensäureester mit Kohlenmonoxid und Alkanolen weiter zu Adipinsäureester umsetzt. Bei der technischen Durchführung eines solchen Verfahrens ist es jedoch erforderlich, den Katalysator wiederzugewinnen und zurückzuführen. So wird in dem aus der US—PS 3 778 466 bekanntgewordenen Verfahren der Katalysator enthaltende Rückstand nach Abdestillieren der Wertprodukte wieder für die Carbonylierung verwendet. Es hat sich jedoch gezeigt, daß beispielsweise nach viermaliger Verwendung die Wirksamkeit des Katalysators erheblich nachläßt.

Es wurde auch schon vorgeschlagen, nach einer Behandlung mit Oxidationsmitteln aus dem Reaktionsgemisch wiedergewonnene Kobaltsalzlösungen gegebenenfalls nach Behandeln mit Ionenaustauschern wiederzuverwenden. Es hat sich durch die Art der Verunreinigungen jedoch gezeigt, daß das Verfahren bei der mehrfachen Recyclisierung der Kobaltsalzlösung verbesserungsbedürftig ist. Durch Anreicherung von Dicarbonsäuren und Stickstoffbasen, z.B. Umwandlungsprodukten des Pyridins, kommt es zu Ausscheidungen und zur Bildung von schwerlöslichen Ölen. Hierdurch wird ein Teil des Kobalt-katalysators abgeschieden, wodurch Verlegungen von Rohrleitungen eintreten. Solche Verbindungen können nur schwer wieder in katalytisch aktive Verbindungen übergeführt werden.

Es war deshalb die technishe Aufgabe gestellt, das Verfahren so zu gestalten, daß die Kobaltkatalysatoren auch nach mehrmaliger Wiederverwendung eine hohe katalytische Aktivität beibehalten, keine Anreicherung an Nebenprodukten eintritt und die damit verbundenen Schwierigkeiten eliminiert werden.

Diese Aufgabe wird gelöst in einem Verfahren zur Herstellung von Butandicarbonsäureestern bei dem man

a) eine wäßrige Kobaltsalzlösung bei Temperaturen von 50 bis 200°C und unter Drücken von 50 bis 500 bar mit überschüssigem Kohlenmonoxid und Wasserstoff in Gegenwart von Aktivkohle, die mit Kobaltcarbonyl beladen ist, behandelt,

b) die erhaltene wäßrige Lösung von Kobaltcarbonylwasserstoff mit Butadien oder Butadien enthaltenden Kohlenwasserstoffgemischen extrahiert und die wäßrige Phase abtrennt,

c) das Kobaltcarbonylhydrid, Kobaltcarbonyl und Butenylkobalttricarbonyl enthaltende Butadien oder Butadien-Kohlenwasserstoffgemisch mit Kohlenmonoxid und $C_1$- bis $C_4$-Alkanolen im Überschuß in Gegenwart von 0,5 bis 2 Mol tertiäre Stickstoffbasen je Mol Butadien mit einem $Pk_a$-Wert von 3 bis 11 bei Temperaturen von 80 bis 150°C und unter Drücken von 300 bis 2000 bar umsetzt,

d) aus dem erhaltenen Reaktionsgemisch die darin enthaltenen tertiären Stickstoffbasen bis auf 0,1 bis 0,3 Mol je Mol Pentensäureester sowie überschüssige Kohlenwasserstoffe abtrennt, den im Reaktionsgemisch verbleibenden Pentenester mit Kohlenmonoxid und $C_1$- bis $C_4$-Alkanolen im Überschuß bei Temperaturen von 140 bis 200°C und unter Drücken von 100 bis 400 bar in Gegenwart der im Reaktionsgemisch vorhandenen Mengen an Kobaltcarbonyl und tertiären Stickstoffbasen umsetzt und anschließend überschüssige Alkanole und freie Stickstoffbasen abdestilliert und

e) das verbleibende Kobaltkatalysatoren Butandicarbonsäureester und Nebenprodukte enthaltende Reaktionsgemisch mit Oxidationsmitteln in Gegenwart der wäßrig sauren Lösung, die in Stufe b) abgetrennt wurde, behandelt und das Gemisch in eine organische Phase, aus der durch Destillation Butandicarbonsäureester gewonnen werden, und eine Kobaltsalze enthaltende wäßrige Phase, die in die Stufe a) zurückgeführt wird, trennt, wobei man die in Stufe b) abgetrennte wäßrige Phase vor der Verwendung in Stufe e) unter erhöhtem Druck auf eine Temperatur von 250 bis 300°C erhitzt.

Das neue Verfahren hat den Vorteil, daß die Katalysatorlösung ohne nachteilige Folgen wiederverwendbar ist. Insbesondere wird die Abscheidung von unlöslichen oder die Bildung von schwer in den aktiven Katalysator überführbare Verbindungen weitgehend vermieden. Ferner hat das neue Verfahren den Vorteil, daß die hierzu nötige Behandlung technisch einfach ist und keiner aufwendigen Hilfsmittel bedarf. Darüber hinaus hat das neue Verfahren den Vorteil, daß die Nebenprodukte in leicht durch Destillation entfernbare Produkte übergeführt und ausgeschleust werden und bei der Verwendung von Pyridin als Stickstoffbase ein Teil der unerwünschten Verbindungen im Pyridin zurückgebildet werden.

In einer ersten Stufe (Stufe a) werden wäßrige Kobaltsalzlösungen mit Kohlenmonoxid und Wasserstoff im Überschuß bei Temperaturen von 50 bis 200°C und unter Drücken von 50 bis 500 bar in Gegenwart von Aktivkohle, die mit Kobaltcarbonyl beladen ist, behandelt. Bevorzugt verwendet man als Kobaltsalze fettsaure Salze, die Wasserlöslich sind, insbesondere Formiate, Acetate, Propionate oder Butyrate. Besonders bewährt haben sich Kobaltformiat und -acetat. Zweckmäßig geht man von Lösungen aus, die 0,5 bis 5 Gew.% Kobalt, berechnet als Metall, insbesondere 1 bis 3 Gew.% Kobalt in Form der genannten Salze enthalten. Im allgemeinen enthält das genannte Gasgemisch Kohlenmonoxid und Wasserstoff im Volumenverhältnis 4:1 bis 1:2, insbesondere im Volumenverhältnis 2:1 bis 1:1. Besonders bewährt hat sich ein annähernd äquimolekulares Gemisch aus Kohlenmonoxid und Wasserstoff. Vorteilhaft verwendet man das Gemisch aus Kohlenmonoxid und Wasserstoff in einem Überschuß, z.B. bis zu dem 5-fachen der stöchiometrisch erforderlichen Menge. Es hat sich bewährt, Temperaturen von 100 bis 170°C und Drücke von 100 bis 400 bar einzuhalten.

Die Behandlung in der Stufe a) erfolgt in Gegenwart von Aktivkohle. Geeignete Aktivkohlearten sind z.B. Torfkohle, Tierkohle oder Zuckerkohle. Als besonders geeignet hat sich Torfkohle erwiesen. Die Aktivkohle ist zweckmäßig bis zu deren Sättigung mit Kobaltcarbonyl beladen. Dies wird im allgemeinen dadurch erreicht, daß man wäßrige Lösungen von Kobaltsalzen zusammen mit dem genannten Gasgemisch aus Kohlenmonoxid und Wasserstoff unter den angegebenen Reaktionsbedingungen über die Aktivkohle bis zu deren Sättigung leitet, d.h. bis man im Ausgang Kobaltcarbonyl und Kobaltcarbonylwasserstoff analytisch feststellt.

Im allgemeinen führt man die Behandlung in einer sogenannten Behandlungszone durch, die zweckmäßig ein Verhältnis von Länge zu Durchmesser von 5 bis 50:1 hat, in der die Aktivkohle in der Regel fest angeordnet ist. Vorzugsweise hält man eine Belastung von 1,5 bis 15 g Kobalt, berechnet als Metall in Form der genannten Salze pro Stunde je Kilogramm Aktivkohle ein.

Die so erhaltene Kobaltcarbonylwasserstoff, nicht umgesetzte Kobaltsalze und freigesetzte Säure enthaltende wäßrige Lösung wird zweckmäßig zusammen mit dem nicht verbrauchten Gemisch aus Kohlenmonoxid und Wasserstoff vorteilhaft ohne Entspannen der zweiten Stufe (Stufe b) zugeführt. Dort wird Kobaltcarbonylwasserstoff mit Butadien oder Butadien enthaltenden Kohlenwasserstoffgemischen, die nachfolgend noch näher erläutert werden, extrahiert. Es ist möglich, die gesamte für die Carbonylierung erforderliche Butadienmenge zur Extraktion zu verwenden oder nur einen Teil derselben. Vorteilhaft verwendet man 5 bis 30 Mol Butadien pro gAtom zu extrahierendes Kobalt. Die Extraktion wird im Gegenstrom oder Gleichstrom in Vorrichtungen durchgeführt, die in der Technik für die Extraktion eingeführt sind, beispielsweise Kolonnen oder statische Mischer.

Während der Extraktion hält man Temperaturen von 20 bis 100°C und Drücke von 5 bis 300 bar ein. Das Gemisch wird anschließend in eine wäßrige Phase und eine organische Phase getrennt. Führt man die Extraktion beispielsweise in einem mit Raschig-Ringen gefüllten Druckrohr durch, so tritt im oberen Teil gleichzeitig eine Trennung in eine organische und eine wäßrige Phase ein. Gleichzeitig wird das mitverwendete Gemisch aus Kohlenmonoxid und Wasserstoff als Gasphase abgetrennt. Der Kobaltgehalt der die Stufe b) verlassenden organischen Phase beträgt im allgemeinen von 1 bis 5 Gew.%. Es wird angenommen, daß das Kobaltcarbonyl als Komplexverbindung mit Butadien, die im Wasser unlöslich ist, in der organischen Phase vorliegt.

Die abgetrennte wäßrige Phase enthält noch bis zu 1 Gew.% $Co^{+2}$ in Form des angewendeten Salzes sowie bis zu 4 Gew.% freie Säure entsprechend dem angewandten Kobaltsalz.

Die organische Phase wird dann in der Stufe c) in Gegenwart von 0,5 bis 2 Mol tertiärer Stickstoffbasen je Mol Butadien mit einem $Pk_a$-Wert von 3 bis 11 mit der Maßgabe, daß die tertiäre Stickstoffbase vorzugsweise niedriger sieden soll als der jeweils herzustellende Pentensäureester mit Co und $C_1$- bis $C_4$-Alkanolen im Überschuß vorteilhaft 1,5 bis 4 Mol je Mol Butadien bei Temperaturen von 80 bis 150°C und unter Drücken von 300 bis 2000 bar umgesetzt.

Falls für die Extraktion nicht die gesamte Menge an Butadien oder solches enthaltendes Kohlenwasserstoffgemisch, das für die Carbonylierung benötigt wird, verwendet wurde, setzt man die erforderliche Menge an Ausgangsstoffen in der Stufe c) zusätzlich zu. Es sei hier vermerkt, daß anstatt reinem Butadien vorteilhaft Butadien enthaltende Kohlenwasserstoffgemische verwendet werden können. Solche Kohlenwasserstoffgemische enthalten neben Butadien gesättigte Kohlenwasserstoffe mit 3 bis 5 Kohlenstoffatomen und einfach olefinisch ungesättigte Kohlenwasserstoffe mit 3 bis 5 Kohlenstoffatomen. Der Butadiengehalt soll in der Regel mehr als 10 Gew.% betragen. In der Technik werden insbesondere $C_4$-Schnitte als Ausgangsgemisch verwendet. Als solche seien alle Gemische von überwiegend unverzweigten $C_4$-Kohlenwasserstoffen definiert, die mehr als 10 Gew.% Butadien-1,3 (Butadien) und mehr 15 Gew.%

Butene enthalten. In der Regel sind solche C$_4$-Schnitte wie folgt zusammengesetzt:

Butadien 40 bis 60 Gew.%
Isobuten 20 bis 35 Gew.%
But-1-en 10 bis 25 Gew.%
But-2-en 5 bis 15 Gew.%
Butane 1 bis 10 Gew.%
Butine 0,1 bis 3 Gew.%.

Solche C$_4$-Schnitte fallen beispielsweise an bei der Dehydrierung von Butan oder Buten oder als Nebenprodukte bei der Äthylengewinnung durch thermische Spaltung von Leichtbenzin oder höhere Kohlenwasserstoffschnitten.

Geeignete tertiäre Stickstoffbasen sind vorzugsweise N-heterocyclische Verbindungen wie Pyridin (Pk$_a$ 5,3), Methylpyridine wie 3-Pikolin (Pk$_a$ 6,0), Isochinolin (Pk$_a$ 5,4), ferner Trialkylamine wie Trimethylamin (Pk$_a$ 9,8) oder Triäthylamin (Pk$_a$ 11,0). Besondere technische Bedeutung hat Pyridin erlangt.

Besonders bewährt hat es sich 0,6 bis 1,5 Mol tetriäre Stickstoffbasen je Mol Butadien anzuwenden.

Als geeignete C$_1$- bis C$_4$-Alkanole seien genannt: methanol, Äthanol, Propanol, Butanol oder Isobutanol. Besonders bevorzugt wird Methanol verwendet.

Die Umsetzung führt man vorzugsweise bei Temperaturen von 120 bis 140°C und Drücken von 600 bis 1200 bar durch. Je Mol Butadien verwendet man in der Regel 0,01 bis 0,1 gAtom Kobalt in Form der beschriebenen Kobaltcarbonylkomplexe an.

Das so erhaltene Reaktionsgemisch enthält neben nicht umgesetztem Butadien gegebenenfalls andere Kohlenwasserstoffe, teriäre Stickstoffbasen, Kobaltcarbonylverbindungen, nicht umgesetzte Alkanole, die als Wertprodukte erzeugten Pentensäureester sowie Nebenprodukte wie Valeriansäureester, Vinylcyclohexen, Butenyl und Butylketone sowie Polymerisate des Butadiens.

Aus dem so erhaltenen Reaktionsgemisch werden nach dem Entspannen die darin enthaltenen tertiären Stickstoffbasen bis auf 0,1 bis 0,3 Mol je Mol Pentensäureester sowie gegebenenfalls überschüssige Kohlenwasserstoffe abgetrennt (Stufe d)). Die Abtrennung erfolgt zweckmäßig durch Destillation. Vorteilhaft wendet man hierbei verminderten Druck an. Insbesondere soll die Temperatur im Destillationssumpf 75°C nicht überschreiten, um eine Zersetzung des Kobaltkatalysators zu vermeiden. Je nach Wahl des mitverwendeten Alkanols destilliert gleichzeitig auch ein Teil oder das gesamte überschüssige Alkanol ab.

Der im Reaktionsgemisch verbleibende Pentensäureester wird mit Kohlenmonoxid und C$_1$- bis C$_4$-Alkanolen im Überschuß, nachdem gegebenenfalls eine entsprechende Menge Alkanole erneut zugegeben wurde, bei Temperaturen von 140 bis 200°C und unter Drücken von 100 bis 400 bar in Gegenwart der im Reaktionsgemisch vorhandenen Menge an Kobaltkatalysator und tertiärer Stickstoffbase umgesetzt. Vorteilhaft hält man Temperaturen von 150 bis 180°C ein. Die mitverwendete Menge an Alkanolen beträgt vorteilhaft 1,5 bis 4 Mol je Mol Pentensäureester. Es hat sich ferner als vorteilhaft erwiesen, dem Kohlenmonoxid einige Volumenprozent Wasserstoff, z.B. 1 bis 4 Vol.% zuzugeben, um die Reaktionsgeschwindigkeit zu erhöhen. Nach dem Entspannen werden aus dem erhaltenen Reaktionsgemisch das überschüssige Alkanol und die freien tertiären Stickstoffbasen abdestilliert. Die chemisch gebundenen tertiären Stickstoffbasen (1 bis 2 Mol/gAtom Kobalt) werden dabei nicht abdestilliert. Um eine Zersetzung des Kobaltcarbonylkomplexes unter Ausscheidung von Kobaltmetall zu vermeiden, die unerwünscht ist, hat es sich bewährt, in den Sumpf der Kolonne einen langsamen Strom von Kohlenmonoxid oder Kohlenmonoxid enthaltenden Gasen einzuleiten.

Das verbleibende Katalysator Butandicarbonsäureester und Nebenprodukte enthaltende Reaktionsgemisch wird in Stufe e) mit Oxidationsmitteln in Gegenwart der wäßrig sauren Lösung, die in Stufe b) abgetrennt wurde, behandelt.

Erfindungsgemäß wird die in Stufe b) abgetrennte wäßrige Phase vor der Verwendung in Stufe e) unter erhöhtem Druck auf eine Temperatur von 250 bis 300°C erhitzt. In der Regel hält man Drücke von 40 bis 300 bar, insbesondere autogenen Druck ein. Es ist darauf zu achten, daß genügend freie Carbonsäure, z.B. 3 bis 4 Gew.% in der wäßrigen Lösung enthalten sind, um eine Ausscheidung von metallischem Kobalt zu vermeiden.

Vorteilhaft führt man die thermische Behandlung über einen Zeitraum von 0,5 bis 3 Stunden durch. Die Behandlung kann absatzweise erfolgen, wird jedoch zweckmäßig kontinuierlich durchgeführt. Um ein Ansteigen des Gehalts an unerwünschten Nebenprodukten zu vermeiden, ist es nicht notwendig, die gesamte Menge der wäßrigen Phase zu behandeln. Vorzugsweise wird nur ein Teilstrom derselben von 5 bis 50 Vol.% der thermischen Behandlung unterworfen und dann mit dem restlichen Teil der nicht behandelten wäßrigen Lösung vereinigt und der Stufe e) zugeführt.

Als Oxidationsmittel sind insbesondere solche geeignet, die das Reaktionsgemisch nicht verunreinigen, beispielsweise Wasserstoffperoxid oder molekularen Sauerstoff enthaltende Gase, insbesondere Luft. Das Oxidationsmittel wird mindestens in einer Menge von 2 Oxidationsäquivalenten je Mol Kobaltverbindung, vorteilhaft jedoch im Überschuß verwendet. In der Praxis hat es sich als zweckmäßig erwiesen, 30 bis 300 N1 Luft je Kilogramm Reaktionsgemisch anzuwenden.

Der sauren wäßrigen Lösung können zusätzlich geeignete Fettsäuren zugesetzt werden, falls erforderlich. Auf jeden Fall ist darauf zu achten, daß genügend Säure vorhanden ist, um den Kobalt in Lösung zu halten. Um die Kobaltlösung nicht zu großer Verdünnung zu erhalten, ist es zweckmäßig, die wäßrige kobalthaltige Lösung im Kreis in den Behandlungsraum zurückzuführen und nur einen kleinen Teilstrom, der der zugeführten Menge entspricht, abzutrennen.

Die oxidative Behandlung erfolgt vorteilhaft bei Temperaturen von 80 bis 160°C. Besonders bewährt haben sich Temperaturen von 100 bis 130°C. Je nach Grad der Durchmischung ist die Umsetzung bereits nach wenigen Sekunden, vielfach bereits innerhalb von Bruchteilen einer Sekunde beendet. Um eine gute Durchmischung zu gewährleisten, ist es angezeigt, das Reaktionsgemisch unter gleichzeitiger Zuführung des Oxidationsmittel in feiner Verteilung der wäßrig sauren Lösung zuzuführen.

Nach der Behandlung trennt man die Flüssigphase, z.B. durch Dekantieren in eine organische Phase und eine wäßrige Phase. Aus der organischen Phase erhält man durch fraktionierte Destillation restliches Pyridin, nicht umgesetzten Pentensäureester, die wieder der Carbonylierung zugeführt werden sowie ein Gemisch aus Butandicarbonsäureestern (80 bis 85 Gew.% Adipinsäureester, 11 bis 15 Gew.% 2-Methylglutarsäureester und 3 bis 6 Gew.% 2-Äthylbernsteinsäureester).

Die Kobaltsalze gegebenenfalls etwas freie Säure enthaltende wäßrige Phase wird vorteilhaft mit Wasser nicht mischbaren Lösungsmitteln, beispielsweise Kohlenwasserstoffen wie Hexan und Cyclohexan, Pentan oder $C_4$-Schnitt extrahiert und der organische Extrakt dem Reaktionsgemisch in e) vor der Phasentrennung zugegeben. Die wäßrige Phase wird wieder in die Stufe a) als Ausgangslösung zur Herstellung von Kobaltcarbonylwasserstoffen zurückgeführt.

Die nach dem Verfahren der Erfindung hergestellten Butandicarbonsäureester eigenen sich zur Herstellung von Diolen oder Polyestern. Der aus dem Estergemisch durch fraktionierte Destillation erhältliche Adipinsäureester eignet sich zur Herstellung von Adipinsäure, Nylonsalz, Adipodinitril oder Hexandiol, d.h. Faservorprodukten.

Das Verfahren nach der Erfindung sei in folgenden Beispielen veranschaulicht.

### Vergleichsbeispiel

Ein Hochdruckrohr (Stufe a)), das mit 600 ml Aktivkohle (Fa. Norit, Körnung 3 bis 5 mm) gefüllt ist, wird stündlich mit 180 ml einer wäßrigen Kobaltacetatlösung, die 2,5 Gew.% $Co^{2+}$ enthält, beschickt. Außerdem führt man stündlich 50 Nl eines äquimolaren Gemisches aus Kohlenmonoxid und Wasserstoff zu. Hierbei hält man eine Temperatur von 120°C und einen Druck von 300 bar ein. Die im anderen Teil des Rohres abgezogene Lösung enthält 0,65 Gew.% $Co^{2+}$ und 1,85 Gew.% Kobalt als Kobaltcarbonylwasserstoff, ferner die entsprechend Menge an Essigsäure. Diese Lösung wird nach dem Entspannen auf 20 bar bei Raumtemperatur intensiv mit 310 ml eines $C_4$-Schnittes, der 43 Gew.% Butadien (1,57 Mol) enthält, gemischt (Stufe b)). Nach der Phasentrennung enthält der $C_4$-Schnitt 3,7 g Kobalt in Form von Kobaltcarbonylverbindungen. Die abgetrennte wäßrige Phase, die später zur Entkobaltung des Reaktionsgemisches verwendet wird, enthält 0,65 Gew.% $Co^{+2}$ und 1,3 Gew.% Essigsäure. Dieser Kobalt enthaltende $C_4$-Schnitt wird nun einem Hochdruckgefäb (Stufe c)) von 1,9 l Inhalts zugeführt und außerdem stündlich 127 ml (1,57 Mol) Pyridin, 127 ml Methanol (3, 14 Mol) und 60 Nl Kohlenmonoxid zugegeben. Die Carbonylierung verläuft bei einer Temperatur von 130°C und 600 bar. Das im Kopf des Hochdruckgefäßes abgenommene Produkt wird entspannt, wobei neben überschüssigem Kohlenmonoxid überschüssige $C_4$-Kohlenwasserstoffe abgetrennt werden. Diese enthalten praktisch kein Butadien. Der Umsatz ist also quantitativ. Unter vermindertem Druck, um den Katalysator zu schonen, werden von dem Austrag stündlich etwa 52 g Methanol und 100 g Pyridin abdestilliert (Stufe d)). Im Destilliationssumpf hält man eine maximale Temperatur von 65°C ein. Dieser Destillationssumpf, der 3,7 g Kobalt als Carbonylkomplex, 165 g (1,44 Mol) Pentensäureester enthält, wird zusammen mit 117 ml (2,88 Mol) Methanol und 55 Nl Kohlenmonoxid, das 2 Vol.% Wasserstoff enthält, kontinuierlich einem weiteren Hochdruckgefäß von 1,7 l Volumen kontinuierlich von unten zugeführt. Die Carbonylierung wird bei einer Temperatur von 170°C und unter einem Druck von 150 bar durchgeführt. Aus dem Austrag (328 g) wird in einer weiteren Kolonne unter Einleiten von etwa 50 Nl Kohlenmonoxid pro Stunde das überschüssige Methanol und das freie Pyridin abdestilliert. Der Destillationssumpf (265 g/Stunde) wird mit 200 ml pro Stunde der in der Extraktionsstufe für Kobaltcarbonyl anfallenden essigsauren wäßrigen Lösung unter Durchleiten von etwa 50 Nl Luft bei 100°C in einem mit Raschig-Ringen gefüllten Rohr gut durchmischt (Stufe e)). Nach der Trennung werden 200 ml wäßrige Kobaltacetatlösung erhalten, die 2,45 Gew.% Kobalt$^{2+}$ enthält, und die nach dem Extrahieren mit Cyclohexan der Kobaltcarbonylbildung in der Stufe a) zugeführt wird. Die organische Phase wird durch fraktionierte Destillation in Pyridin (etwa 5 g), Valeriansäureester (6,5 g), Pentenester (11,5 g) und Butandicarbonsäuredimethylester (220 g) aufgetrennt. In den Butandicarbonsäuredimethylestern sind 181 g Adipinsäuredimethylester enthalten.

Nach 25-maliger Rückführung der wäßrigen Kobaltsalzlösung findet man in der wäßrigen Phase nach Stufe b) 0,5 Gew.% Kobalt als Kobaltacetat, 3 Gew.% Essigsäure, 1,3 Gew.% Adipinsäuremonomethylester, 1,5 Gew.% Adipinsäure und 1,2 Gew.% Stickstoffbasen (N-Methylpyridin, N-Methyl-2-dihydropyridin und höhermolekulare N-Basen).

Führt man die Rückführung fort, so scheiden sich bei 40°C aus der wäßrigen Lösung schwerlösliche Salze ab.

### Beispiel 1

Man verfährt wie im Vergleichsbeispiel beschrieben, erhitzt jedoch nach 25-maliger Rückführung die in Stufe b) abgetrennte wäßrige Phase vor der Verwendung in Stufe e) 2 Stunden auf 300°C bei 100 bar unter Eigendruck. In der so behandelten Lösung wird Adipinsäuremonomethylester quantitativ, Adipinsäure zu 93% und die Stickstoffbasen zu 75% zersetzt. Die so behandelte Lösung enthält nunmehr 0,5 Gew.% Kobalt als Acetat, 3 Gew.% Essigsäure, 0,1 Gew.% Adipinsäure und 0,3 Gew.% Stickstoffbasen. Nach einer weiteren 25-maligen Rückführung wird die Behandlung wiederholt. Es sind keine Abscheidungen festzustellen.

**Patentansprüche**

1. Verfahren zur Herstellung von Butandicarbonsäureestern, bei dem man

a) eine wäßrige Kobaltsalzlösung bei Temperaturen von 50 bis 200°C und unter Drücken von 50 bis 500 bar mit überschüssigem Kohlenmonoxid und Wasserstoff in Gegenwart von Aktivkohle, die mit Kobaltcarbonyl beladen ist, behandelt,

b) die erhaltene wäßrige Lösung von Kobaltcarbonylwasserstoff mit Butadien oder Butadien enthaltenden Kohlenwasserstoffgemischen extrahiert und die wäßrige Phase abtrennt,

c) das Kobaltcarbonylhydrid, Kobaltcarbonyl und Butenylkobalttricarbonyl enthaltende Butadien oder Butadien-Kohlenwasserstoffgemisch mit Kohlenmonoxid und $C_1$- bis $C_4$-Alkanolen im Überschuß in Gegenwart von 0,5 bis 2 Mol tertiäre Stickstoffbasen je Mol Butadien mit einem $Pk_a$-Wert von 3 bis 11 bei Temperaturen von 80 bis 150°C und unter Drücken von 300 bis 2000 bar umsetzt,

d) aus dem erhaltenen Reaktionsgemisch die darin enthaltenen tertiären Stickstoffbasen bis auf 0,1 bis 0,3 Mol je Mol Pentensäureester sowie überschüssige Kohlenwasserstoffe abtrennt, den im Reaktionsgemisch verbleibenden Pentensäureester mit Kohlenmonoxid und $C_1$- bis $C_4$-Alkanolen im Überschuß bei Temperaturen von 140 bis 200°C und unter Drücken von 100 bis 400 bar in Gegenwart der im Reaktionsgemisch vorhandenen Mengen an Kobaltcarbonyl und tertiären Stickstoffbasen umsetzt und anschließend überschüssige Alkanole und freie Stickstoffbasen abdestilliert und

e) das verbleibende Kobaltkatalysator Butandicarbonsäureester und Nebenprodukte enthaltende Reaktionsgemisch mit Oxidationsmitteln in Gegenwart der wäßrig sauren Lösung, die in Stufe b) abgetrennt wurde, behandelt und das Gemisch in eine organische Phase, aus der durch Destillation Butandicarbonsäureester gewonnen werden, und eine Kobaltsalze enthaltende wäßrige Phase, die in die Stufe a) zurückgeführt wird, trennt, dadurch gekennzeichnet, daß man die in Stufe b) abgetrennte wäßrige Phase vor der Verwendung in Stufe e) unter erhöhtem Druck auf eine Temperatur von 250 bis 350°C erhitzt.

2. Verfahren nach Anspruch 1, Dadurch gekennzeichnet, daß man autogenen Druck anwendet.

3. Verfahren nach den Ansprüchen 1 und 2, dadurch gekennzeichnet, daß man einen Teilstrom von 5 bis 50 Vol.% der in b) anfallenden wäßrigen Phase behandelt und zusammen mit dem unbehandelten Teil der Stufe e) zuführt.

**Claims**

1. Process for the preparation of butanedicarboxylic acid esters, wherein

a) an aqueous cobalt salt solution is treated, at from 50 to 200°C and under a pressure of from 50 to 500 bar, with excess carbon monoxide and hydrogen in the presence of active charcoal laden with cobalt carbonyl,

b) the resulting aqueous solution of cobalt carbonyl hydride is extracted with butadiene, or with a hydrocarbon mixture containing butadiene, and the aqueous phase is separated off,

c) the butadiene, or the butadiene/hydrocarbon mixture, containing cobalt carbonyl hydride, cobalt carbonyl and butenyl-cobalt tricarbonyl, is reacted with carbon monoxide and a $C_1$—$C_4$-alkanol in excess in the presence of from 0.5 to 2 moles, per mole of butadiene, of a tertiary nitrogen base having a $pK_a$ of from 3 to 11, at from 80 to 150°C and under a pressure of from 300 to 2,000 bar,

d) the tertiary nitrogen base contained in the resulting reaction mixture is removed until its content reaches 0.1 to 0.3 mole per mole of pentenoic acid ester, excess hydrocarbons are also removed, the pentenoic acid ester remaining in the reaction mixture is reacted with carbon monoxide and a $C_1$—$C_4$-alkanol in excess at from 140 to 200°C and under a pressure of from 100 to 400 bar in the presence of the amounts of cobalt carbonyl and tertiary nitrogen base present in the reaction mixture, and excess alkanol and free nitrogen base are then distilled off, and

e) the reaction mixture which is left and which contains a cobalt catalyst, butanedicarboxylic acid ester and by-products is treated with oxidizing agents in the presence of the aqueous acid solution which has been removed in stage b), and the mixture is separated into an organic phase, from which butanedicarboxylic acid ester is isolated by distillation, and an aqueous phase, containing cobalt salts, which is recycled to stage a), characterized in that the aqueous phase separated off in stage b) is heated at from 250 to 350°C under superatmospheric pressure, before being used in stage e).

2. A process as claimed in claim 1, wherein autogenous pressure is used.

3. A process as claimed in claim 1 or 2, wherein a part-stream of from 5 to 50% by volume of the aqueous phase obtained in b) is treated and is fed, together with the untreated part, to stage e).

## Revendications

1. Procédé de préparation d'esters d'acides butanedicarboxyliques dans lequel

a) on traite une solution aqueuse de sel de cobalt avec de l'oxyde de carbone en excès et de l'hydrogène à des températures comprises entre 50 et 200°C et sous des pressions variant entre 50 et 500 bars, en présence de charbon actif chargé de cobalt carbonyle,

b) on extrait la solution aqueuse d'hydrure de cobalt carbonyle ainsi obtenue avec du butadiène ou des mélanges d'hydrocarbures contenant du butadiène et on sépare la phase aqueuse,

c) on fait réagir le butadiène, ou le mélange d'hydrocarbures/butadiene, renfermant de l'hydrure de cobalt, du cobalt carbonyle et du buténylcobalt tricarbonyle, avec de l'oxyde de carbone et des alcanols en excès en $C_1$ à $C_4$, en présence de 0,5 à 2 moles de bases d'azote tertiares par mole de butadiène, d'un indice $pK_a$ de 3 à 11, à des températures comprises entre 80 et 150°C et sous des pressions variant entre 300 et 2000 bars,

d) on sépare du mélange réactionnel ainsi obtenu les bases d'azote tertiares qu'il renferme jusqu'à un reste de 0,1 à 0,3 mole par mole d'ester d'acide penténique, ainsi que les hydrocarbures en excès, on fait réagir l'ester d'acide penténique restant dans le mélange réactionnel avec de l'oxyde de carbone et des alcanols en excès en $C_1$ à $C_4$, à des températures comprises entre 140 et 200°C et sous des pressions variant entre 100 et 400 bars, en présence des quantités de cobalt carbonyle et de bases d'azote tertiaires contenues dans le mélange réactionnel, et on sépare ensuite par distillation les alcanols en excès et les bases d'azote libres, et

e) on traite le mélange réactionnel restant renfermant le catalyseur de cobalt, des esters d'acides butanedicarboxyliques et des sous-produits par des oxydants en présence de la solution aqueuse acide séparée dans le stade b), et on sépare le mélange en une phase organique à partir de laquelle on obtient par distillation des esters d'acides butanedicarboxyliques, et en une phase aqueuse contenant des sels de cobalt que l'on ramène au stade a),

ce procédé étant caractérisé par le fait que, avant de l'amener au stade e), on chauffe la phase aqueuse séparée dans le stade b) à une température comprise entre 250 et 350°C, sous pression élevée.

2. Procédé selon la revendication 1, dans lequel on opère sous pression autogène.

3. Procédé selon l'une des revendications 1 ou 2, dans lequel on traite un courant partiel de 5 à 50% en volume de la phase aqueuse formée dans le stade b) et on l'amène au stade e) simultanément avec la partie de la phase aqueuse non traitée.